## Europäisches Patentamt
## European Patent Office
## Office européen des brevets

(19)

(11) Numéro de publication: **0 210 694**
**B1**

(12) # FASCICULE DE BREVET EUROPÉEN

(45) Date de publication du fascicule du brevet:
08.03.89

(51) Int. Cl.⁴: **C 07 C 19/05,** C 07 C 17/42

(21) Numéro de dépôt: **86201230.9**

(22) Date de dépôt: **15.07.86**

(54) Compositions stabilisées de 1,1,1-trichloréthane.

(30) Priorité: **25.07.85 FR 8511531**

(43) Date de publication de la demande:
**04.02.87 Bulletin 87/6**

(45) Mention de la délivrance du brevet:
**08.03.89 Bulletin 89/10**

(84) Etats contractants désignés:
**AT BE CH DE FR GB IT LI LU NL SE**

(56) Documents cité:
**EP-A-0 044 111**
**FR-A-2 268 773**
**US-A-3 281 480**

(73) Titulaire: **SOLVAY & Cie (Société Anonyme), Rue du Prince Albert, 33, B-1050 Bruxelles (BE)**

(72) Inventeur: **Servais, Michel, Chaussée de Malines, 315, B-1950 Kraainem (BE)**
Inventeur: **Verbeyst, Jean, Termolenhoflaan, 46, B-1730 Zellik (BE)**

LIBER, STOCKHOLM 1989

EP 0 210 694 B1

## Description

La présente invention concerne des compositions stabilisées de 1,1,1-trichloréthane, utilisables notamment pour le dégraissage des métaux et pour le nettoyage à sec des textiles.

On sait que le 1,1,1-trichloréthane, qui est utilisé notamment pour le dégraissage des métaux, le nettoyage à sec des textiles, et dans les aérosols, pose un problème de stabilité et de corrosion tout particulier. L'emploi, pour stabiliser le 1,1,1-trichloréthane, des stabilisants habituellement utilisés pour d'autres hydrocarbures chlorés ne donne pas, en général, des résultats satisfaisants. Ce phénomène a été attribué notamment au fait qu'il existe une grande réactivité entre le 1,1,1-trichloréthane et les métaux, tels que l'aluminium, le zinc et leurs alliages, et l'eau. Dès lors, on a développé des compositions stabilisées, spécifiques du 1,1,1-trichloréthane, nombreuses et variées.

Ainsi on a proposé dans les brevets Etats-Unis 3 281 480 et 3 128 315 de Pittsburgh Plate Glass de stabiliser le 1,1,1-trichloréthane au moyen d'un mélange de stabilisants contenant un dialcoxyalcane tel que le 1,2-diméthoxyéthane, un mono-alcool aliphatique tel que l'alcool butylique tertiaire, un nitroalcane tel que le nitrométhane et un époxyde tel que l'époxybutane.

De même on a proposé dans la demande de brevet français 2 241 520 de La Société Chimique des Charbonnages de stabiliser le 1,1,1-trichloréthane au moyen de diméthoxyméthane seul ou en association avec d'autres costabilisants choisis notamment parmi l'alcool butylique tertiaire, l'époxybutane et le nitrométhane.

Les quantités des stabilisants mis en oeuvre sont très élevées en vue d'obtenir une stabilisation satisfaisante.

De plus les alcools présents dans les compositions stabilisées entraînent la formation de taches lors de l'évaporation.

Aucune des compositions proposées jusqu'à ce jour n'est vraiment efficace. En particulier aucune composition connue du 1,1,1-trichloréthane ne présente une stabilité satisfaisante dans des conditions extrêmement sévères telles que celles prévalant lors de la présence simultanée de métaux légers et d'eau et plus particulièrement lorsque l'eau est présente en quantités élevées, supérieures à la valeur saturante à la température et la pression considérées.

La présente invention vise à remédier à ces inconvénients des compositions connues en procurant des compositions stabilisées de 1,1,1-trichloréthane dont la stabilité est satisfaisante même dans les conditions d'utilisation les plus sévères.

L'invention concerne à cet effet des compositions stabilisées de 1,1,1-trichloréthane contenant au moins deux dialcoxyalcanes de formule générale $R_2 - O - R_1 - O - R_3$ dans laquelle $R_1$, $R_2$ et $R_3$ représentent indépendemment les uns des autres des radicaux aliphatiques saturés contenant de 1 à 4 atomes de carbone.

Habituellement $R_1$ représente un radical aliphatique saturé de de formule $-(CH_2)_n$ dans lequel n est un nombre entier de 1 à 4; de manière préférée n est égal à 1 ou 2.

Habituellement $R_2$ et $R_3$ représentent des radicaux aliphatiques saturés, de formule $-(CH_2)_r-CH_3$ dans lesquels r est un nombre entier de 0 à 3; de manière préférée r est égal à 0.

De bons résultats ont été obtenus lorsque les compositions contiennent simultanément du diméthoxyméthane et du 1,2-diméthoxyéthane.

La quantité de chaque dialcoxyalcane présente dans les compositions selon l'invention varie habituellement entre 0,1 et 100 grammes par litre de 1,1,1-trichloréthane. De préférence cette quantité est comprise entre 1 et 75 grammes par litre. Tout particulièrement préférées sont des quantités comprises entre 5 et 50 grammes par litre.

Les quantités des dialcoxyalcanes différents présents dans le mélange peuvent être identiques ou différentes. De bons résultats ont été obtenus lorsque le diméthoxyméthane et le 1,2-diméthoxyéthane sont mis en oeuvre dans des rapports molaires compris entre 0,1 et 10 et de préférence compris entre 0,2 et 5.

En outre les compositions de l'invention comprennent avantageusement au moins un accepteur d'acide tels que des composés époxydés.

Par composé époxydé, on entend les composés aliphatiques saturés ou insaturés contenant au moins un groupement époxyde dans leur molécule. De préférence, les compositions selon l'invention contiennent des composés époxydes aliphatiques saturés contenant de 3 à 6 atomes de carbone dans leur molécule tels que l'époxypropane, l'époxybutane, les 2-méthylépoxypropanes, les 2-méthylépoxybutanes, le glycidol et l'épichlorhydrine. De bons résultats ont été obtenus avec le 2-méthyl-2,3-époxybutane et l'époxybutane qui est tout particulièrement préféré.

La quantité totale de composé époxydé présente dans les compositions selon l'invention varie habituellement entre 0,01 et 50 grammes par litre. De préférence, cette quantité est comprise entre 0,1 et 20 grammes par litre. Tout particulièrement préférées sont des quantités comprises entre 1 et 10 grammes par litre.

Outre les composés précités les compositions selon l'invention peuvent contenir des dérivés nitrés tels que des nitroalcanes contenant de 1 à 4 atomes de carbone dans leur molécule. De bons résultats ont été obtenus avec le nitrométhane, le nitroéthane et les nitropropanes 1 ou 2. Tout particulièrement préférés sont le nitroéthane et surtout le nitrométhane, ainsi que les mélanges de ces deux composés.

Les quantités de dérivés nitrés sont les mêmes que celles définies pour les composés époxydés.

D'autres composés et notamment, les nitrates tels que les nitrates d'alkyle contenant de 1 à 5 atomes de carbone, les amines tels que les amines aliphatiques contenant de 2 à 6 atomes de carbone, les pyrroles, les quinones et d'autres composés aromatiques tels que le phénol et ses dérivés et le toluène ou aliphatiques tels que le diisobutylène peuvent être également incorporés dans la formule de stabilisation du 1,1,1-trichloréthane mise finalement en oeuvre comme costabilisants.

Ces différents stabilisants utilisés sont généralement présents dans des quantités très variables comprises entre 0,001 et 50 g/l de 1,1,1-trichloréthane et de préférence dans des quantités comprises entre 0,1 et 30 g/l.

Les compositions de 1,1,1-trichloréthane stabilisées selon l'invention conviennent particulièrement bien pour les applications requérant simultanément une bonne stabilisation du 1,1,1-trichloréthane considéré et une quantité totale de stabilisants présents aussi faible que possible. Elles sont utilisées avantageusement pour dégraisser les métaux à chaud, à froid ou en phase vapeur et tout particulièrement pour le dégraissage des métaux à froid étant donné que les compositions de 1,1,1-trichloréthane stabilisées selon l'invention présentent la particularité de mouiller très efficacement les pièces métalliques. De plus ces compositions ne contenant pas d'alcool, ne laissent aucune trace lors de l'évaporation.

Les compositions selon l'invention présentent également une excellente aptitude au recyclage.

Les exemples qui suivent n'ont aucun caractère limitatif.

## Exemple 1

Cet exemple est effectué afin de montrer la stabilité améliorée observée avec des compositions selon l'invention (essai 1) en comparaison avec d'autres compositions du même type contenant un seul dialcoxyalcane (essais 2 et 3) dans des conditions opératoires sévères c'est-à-dire en présence de métaux légers et d'eau. Les quantités de produits mis en oeuvre ainsi que les résultats obtenus dans les différents essais ont été rassemblés au tableau I ci-après.

## Essai 1: composition selon l'invention

Dans un appareil Soxhlet comportant un ballon à fond rond de 500 cm$^3$ et un réservoir de 100 cm$^3$ sans cartouche filtrante, dont le chauffage est assuré par un manteau chauffant, on introduit 200 cm$^3$ de 1,1,1-trichloréthane, stabilisé au moyen de 5 g/l de nitrométhane, 5,7 g/l de 1,2-époxybutane, 20 g/l de diméthoxyméthane et 25 g/l de 1,2-diméthoxyéthane.

On introduit ensuite 2 cm$^3$ d'eau déminéralisée.

Tout en maintenant le mélange à température ambiante on introduit ensuite les éprouvettes de 40 x 8 x 3 mm en alliage d'aluminium contenant 4,5 % de cuivre, 0,6 % de magnésium et 0,6 % de manganèse (alliage AU 4 G-T4); une éprouvette est immergée dans le réservoir du Soxhlet et une seconde dans le réfrigérant.

Le ballon est ensuite chauffé de manière à obtenir une vidange du réservoir toutes les trentes minutes (≃ 2 minutes).

Après 72 h d'ébullition on refroidit le ballon à la température de 20°C, on vide le ballon et le réservoir.

On rince soigneusement le ballon, le réservoir, le réfrigérant et les éprouvettes à l'eau déminéralisée et le liquide de rinçage sert à l'extraction du 1,1,1-trichloréthane récupéré.

On détermine l'acceptation d'acide sur la phase organique.

On porte l'extrait aqueux à 250 cm$^3$ dans un ballon jaugé et on dose les ions Cl⁻ formés par mercurimétrie.

Les éprouvettes sont brossées sous eau à l'aide d'une brosse dure non métallique; puis après rinçage à l'eau déminéralisée elles sont séchées à l'acétone, puis pesées à 0,1 mg près. Les résultats observés sont repris au Tableau I ci-après. Chaque essai a été réalisé deux fois.

Par ailleurs aucune trace n'est observée sur ces éprouvettes lors de l'évaporation du 1,1,1-trichloréthane stabilisé.

## Essais 2 et 3: compositions de comparaison

Les essais 2 et 3 sont identiques à l'essai 1 pour les conditions opératoires.

Pour l'essai 2, le 1,1,1-trichloréthane est stabilisé au moyen de 5 g/l de nitrométhane, 5,7 g/l de 1,2-époxybutane et 45 g/l de diméthoxyméthane.

L'essai 3 est réalisé au moyen de 5 g/l de nitrométhane, 5,7 g/l de 1,2-époxybutane et 45 g/l de 1,2-diméthoxyéthane.

**Tableau I**

| Stabilisants | | Essai 1 | | Essai 2 | | Essai 3 | |
| --- | --- | --- | --- | --- | --- | --- | --- |
| g/l | | 1 | 2 | 1 | 2 | 1 | 2 |
| Diméthoxyméthane | | 20 | 20 | 45 | 45 | 0 | 0 |
| 1,2-diméthoxyéthane | | 25 | 25 | 0 | 0 | 45 | 45 |
| Nitrométhane | | 5 | 5 | 5 | 5 | 5 | 5 |
| 1,2-époxybutane | | 5,7 | 5,7 | 5,7 | 5,7 | 5,7 | 5,7 |
| Résultats après test | | | | | | | |
| Perte en acceptation d'acide | % | 9 | 13 | 59 | 77 | 77 | 81 |
| Cl⁻ formés | meq/l | 2,3 | 1 | 63 | 52 | 74 | 56 |
| Perte en poids | | | | | | | |
| des éprouvettes g/m²/jour | Soxhlet | 17 | 0 | 122 | 129 | 167 | 132 |
| | Réfrigérant | 1,4 | 0,1 | 160 | 154 | 220 | 185 |

On peut donc conclure à l'examen des résultats du Tableau I qu'en présence d'eau et d'aluminium, la composition selon l'invention (essai 1), comparée aux autres compositions (essais 2 et 3) contenant un seul dialcoxyalcane est 6 à 8 fois plus efficace du point de vue de l'acceptation d'acide, forme 25 à 75 fois moins d'ions Cl⁻ et corrode 10 à 200 fois moins les éprouvettes en alliage d'aluminium en contact à la fois avec l'eau et le 1,1,1-trichloréthane.

**Exemple 2**

Cet exemple est effectué afin de montrer la stabilité améliorée observée avec des compositions selon l'invention (essai 1) contenant un mélange de diméthoxyméthane, de 1,2-diméthoxyéthane et d'époxybutane en comparaison avec d'autres compositions contenant un seul dialcoxyalcane (diméthoxyméthane), un alcool et de l'époxybutane (essai 2).

**Essai 1:** composition selon l'invention

Dans cet essaï le 1,1,1-trichloréthane est stabilisé au moyen de:

- 30 g/l de diméthoxyméthane,
- 20 g/l de 1,2-diméthoxyéthane et
- 5,7 g/l de 1,2-époxybutane.

On opère dans les mêmes conditions opératoires que décrites dans l'exemple 1, essai 1.
Les résultats obtenus sont les suivants:

- perte en acceptation d'acide: 78 %
- ions Cl⁻ formés 66 meq/l
- perte en poids des éprouvettes en g/m²/jour:
  * Soxhlet: 129
  * réfrigérant: 143

**Essai 2:** compositions de comparaisons

Dans ces essais le 1,1,1-trichloréthane est stabilisé au moyen de:

a)  - 30 g/l de diméthoxyméthane,
    - 20 g/l d'alcool butylique tertiaire et
    - 5,7 g/l de 1,2-époxybutane.
b)  - 30 g/l de 1,2-diméthoxyéthane
    - 20 g/l d'alcool butylique tertiaire et
    - 5,7 g/l de 1,2-époxybutane.

On opère à nouveau dans les mêmes conditions opératoires que décrites dans l'exemple 1, essai 1.
On observe dans les deux cas une décomposition violente.

4

On peut donc conclure de la comparaison des résultats obtenus dans l'essai 1 et l'essai 2 que les compositions de l'invention donnent dans le test utilisé en l'absence de nitrométhane des résultats acceptables, alors qu'une composition ne contenant qu'un seul dialcoxyalcane ne confère en l'absence de nitrométhane aucune stabilité au 1,1,1-trichloréthane.

**Exemple 3**

Cet exemple est effectué afin de montrer que des compositions selon l'invention même en l'absence de nitroalcane satisfont à des tests officiels pour l'agrément de solvants halogénés pour le dégraissage des métaux légers en Allemagne.

**Essai 1:** composition selon l'invention

Dans cet essai le 1,1,1-trichloréthane est stabilisé au moyen de:

- 10 g/l de diméthoxyméthane,
- 31 g/l de 1,2 diméthoxyéthane,
- 5,2 g/l d'époxybutane.

Avec cette composition on réalise le test suivant connu en Allemagne comme le test BAM I (Bundesanstalt für Materialprüfung).
Dans un ballon à fond rond de 500 cm$^3$ muni d'un ajutage latéral permettant la mesure de la température du liquide à l'aide d'un thermocouple, on introduit 100 cm$^3$ de 1,1,1-trichloréthane stabilisé.
On introduit ensuite 100 cm$^3$ de toluène dans le ballon et on mélange le tout.
A ce mélange on ajoute 18 g de paillettes d'aluminium (paillettes de 0,1 à 1 mm de diamètre) et 0,7 g de AlCl$_3$ anhydre, sublimé et sans agglomérat.
Ce mélange est soumis à une ébullition à reflux durant 9 h dans le ballon de 500 cm$^3$ chauffé dans un bain d'huile, thermostatisé à 40°C au dessus de la température d'ébullition du 1,1,1-trichloréthane.
On arrête l'ébullition durant 15 h.
Puis on soumet de nouveau le mélange à une ébullition à reflux durant 21 h.
Le 1,1,1-trichloréthane ne satisfait pas au test BAM I lorsqu'il subit une décomposition exothermique ou lorsqu'il continue à se décomposer après éloignement de la source de chaleur.
La composition stabilisée selon l'invention n'a pas subi de décomposition exothermique durant les 9 h d'ébullition, les 15 h d'arrêt puis les 21 h d'ébullition.

**Essai 2:** composition de comparaison

Dans cet essai le 1,1,1-trichloréthane est stabilisé au moyen de:

- 31 g/l de 1,2-diméthoxyéthane,
- 21 g/l d'alcool butylique tertiaire et
- 5,2 g/l de 1,2-époxybutane.

Cette composition est soumise au même test de stabilité que la composition de l'exemple 3 essai 1.
Cette composition ne subit pas de décomposition exothermique durant les 9 heures d'ébullition et les 15 heures d'arrêt mais se décompose après 10 heures de la seconde ébullition.

**Essai 3:** composition de comparaison

Dans cet essai le 1,1,1-trichloréthane est stabilisé au moyen de:

- 31 g/l de 1,2-diméthoxyéthane,
- 21 g/l de 2-méthyl-3-butène-2-ol,
- 5,2 g/l du 1,2-époxybutane.

Cette composition est également soumise au même test que celles des essais 1 et 2 ci-avant.
Déjà après 5,5 heures d'ébullition cette composition se décompose.

On peut conclure de la comparaison des résultats obtenus dans les essais 1, 2 et 3 ci-avant que la seule composition ne contenant pas de nitrométhane satisfaisant au test officiel BAM I est la composition selon l'invention (essai 1) et cela malgré que la quantité totale de stabilisants soit inférieure à celles des essais 2 et 3.

**Exemple 4**

Cet exemple est effectué afin de montrer que les compositions selon l'invention même en l'absence de nitrométhane satisfont également au test BAM IV officiel sévère utilisé en Allemagne pour l'agrément des solvants halogénés pour le dégraissage des métaux légers.

La composition de 1,1,1-trichloréthane contient

- 20 g/l de diméthoxyméthane,
- 50 g/l de 1,2-diméthoxyéthane et
- 5,7 g/l de 1,2-époxybutane.

On divise 500 cm$^3$ du 1,1,1-trichloréthane stabilisé en trois fractions égales par distillation simple. La fraction de queue n'est pas distillée et la vitesse de distillation est de l'ordre de 20 cm$^3$ par minute.

Sur chaque fraction on effectue maintenant le test décrit dans l'exemple 3 essai 1.

Les trois fractions de la composition ne subissent pas de décomposition durant les 9 heures d'ébullition, les 15 heures d'arrêt et les 21 heures d'ébullition finale. La composition selon l'invention satisfait donc parfaitement au test BAM IV malgré l'absence de nitroalcane.

**Revendications**

1. Compositions stabilisées de 1,1,1-trichloréthane caractérisées en ce qu'elles contiennent au moins deux dialcoxyalcanes de formule générale $R_2 - O - R_1 - O - R_3$ dans laquelle $R_1$, $R_2$ et $R_3$ représentent indépendemment les uns des autres des radicaux aliphatiques saturés contenant de 1 à 4 atomes de carbone.

2. Compositions selon la revendication 1 caractérisées en ce que $R_1$ représente un radical aliphatique saturé de formule $-(CH_2)_n-$ dans lequel n est un nombre entier de 1 à 4 et $R_2$ et $R_3$ représentent des radicaux aliphatiques saturés de formule $-(CH_2)_r-CH_3$ dans lesquels r est un nombre entier de 0 à 3.

3. Compositions selon la revendication 2 caractérisées en ce que l'un des dialcoxyalcanes est le diméthoxyméthane.

4. Compositions selon la revendication 2 caractérisées en ce que l'un des dialcoxyalcanes est le 1,2-diméthoxyéthane.

5. Compositions selon les revendications 3 et 4 caractérisées en ce qu'elles contiennent simultanément le diméthoxyméthane et le 1,2-diméthoxyéthane.

6. Compositions selon les revendications 1 à 5 caractérisées en ce que chaque dialcoxyalcane est présent dans des quantités comprises entre 0,1 et 100 g par litre de 1,1,1-trichloréthane.

7. Compositions selon la revendications 5 caractérisées en ce que le diméthoxyméthane et le 1,2-diméthoxyéthane sont mis en oeuvre dans des rapports molaires compris entre 0,1 et 10.

8. Compositions selon la revendication 1 caractérisées en ce qu'elles comprennent outre les dialcoxyalcanes au moins un composé époxydé et éventuellement un dérivé nitré.

9. Compositions selon la revendication 8 caractérisées en ce que le composé époxydé est le 2-méthyl-2,3-époxybutane ou le 1,2-époxybutane et que le dérivé nitré est le nitrométhane et/ou le nitroéthane.

10. Compositions selon la revendication 8 caractérisées en ce que le composé époxydé et le dérivé nitré sont présents dans des quantités comprises entre 0,01 et 50 g par litre de 1,1,1-trichloréthane.

**Patentansprüche**

1. Stabilisierte Zusammensetzungen von 1,1,1,-Trichlorethan, dadurch gekennzeichnet, daß sie mindestens zwei Dialkoxyalkane der allgemeinen Formel $R_2 - O - R_1 - O - R_3$ enthalten, worin $R_1$, $R_2$ und $R_3$ unabhängige Voneinander aliphatische gesättigte Reste, die 1 bis 4 Kohlenstoffatome enthalten, darstellen.

2. Zusammensetzungen nach Anspruch 1, dadurch gekennzeichnet, daß $R_1$ einen aliphatischen, gesättigten Rest der Formel $-(CH_2)_n-$ darstellt, in dem n eine ganze Zahl von 1 bis 4 ist und $R_2$ und $R_3$ aliphatische, gesättigte Reste der Formel $-(CH_2)_r-CH_3$ darstellen, in denen r eine ganze Zahl von 0 bis 3 ist.

3. Zusammensetzungen nach Anspruch 2, dadurch gekennzeichnet, daß eines der Dialkoxyalkane das Dimethoxymethan ist.

4. Zusammensetzungen nach Anspruch 2, dadurch gekennzeichnet, daß eines der Dialkoxyalkane das 1,2-Dimethoxyethan ist.

5. Zusammensetzungen nach den Ansprüchen 3 und 4, dadurch gekennzeichnet, daß sie gleichzeitig das Dimethoxymethan und das 1,2-Dimethoxyethan enthalten.

6. Zusammensetzungen nach den Ansprüchen 1 bis 5, dadurch gekennzeichnet, daß jedes Dialkoxyalkan in Mengen zwischen 0,1 und 100 g pro Liter 1,1,1-Trichlorethan vorhanden ist.

7. Zusammensetzungen nach Anspruch 5, dadurch gekennzeichnet, daß das Dimethoxymethan und das 1,2-Dimethoxyethan in molaren Verhältnissen zwischen 0,1 und 10 eingesetzt werden.

8. Zusammensetzungen nach Anspruch 1, dadurch gekennzeichnet, daß sie außer den Dialkoxyalkanen mindestens eine epoxidierte Verbindung und eventuell ein nitriertes Derivat umfassen.

9. Zusammensetzungen nach Anspruch 8, dadurch gekennzeichnet, daß die epoxidierte Verbindung das 2-Methyl-2,3-epoxybutan oder das 1,2-Epoxybutan ist und, daß das nitrierte Derivat das Nitromethan und/oder das Nitroethan ist.

10. Zusammensetzungen nach Anspruch 8, dadurch gekennzeichnet, daß die epoxidierte Verbindung und das nitrierte Derivat in Mengen zwischen 0,01 und 50 g pro Liter 1,1,1-Trichlorethan vorhanden sind.

**Claims**

1. Stabilized compositions of 1,1,1-trichloroethane characterized in that they contain at least two dialkoxyalkanes of general formula $R_2 - O - R_1 - O - R_3$ in which $R_1$, $R_2$ and $R_3$ represent, independently from one another, saturated aliphatic radicals containing from 1 to 4 carbon atoms.

2. Compositions according to Claim 1, characterized in that $R_1$ represents a saturated aliphatic radical of formula $-(CH_2)_n$ in which n is an integer of 1 to 4 and $R_2$ and $R_3$ represent saturated aliphatic radicals of formula $-(CH_2)_r-CH_3$ in which R is an integer of 0 to 3.

3. Compositions according to Claim 2, characterized in that one of the dialkoxyalkanes is dimethoxymethane.

4. Compositions according to Claim 2, characterized in that one of the dialkoxyalkanes is 1,2-dimethoxyethane.

5. Compositions according to Claims 3 and 4, characterized in that they contain the dimethoxymethane and the 1,2-dimethoxyethane at the same time.

6. Compositions according to Claims 1 to 5, characterized in that each dialkoxyalkane is present in quantities of between 0.1 and 100 g per titre of 1,1,1-trichloroethane.

7. Compositions according to Claim 5, characterized in that the dimethoxymethane and the 1,2-dimethoxyethane are employed in molar ratios of between 0.1 and 10.

8. Compositions according to Claim 1, characterized in that they additionally contain dialkoxyalkanes, at least one epoxidated compound and, if appropriate, a nitrated derivative.

9. Compositions according to Claim 8, characterized in that the epoxidated compound is 2-methyl-2,3-epoxybutane or 1,2-epoxybutane and that the nitrated derivative is ni tromethane and/or ni troethane.

10. Compositions according to Claim 8, characterized in that the epoxidated compound and the nitrated derivative are present in quantities of between 0.01 and 50 g per litre of 1,1,1-trichloroethane.